# EUROPEAN PATENT APPLICATION

(11) **EP 2 281 808 A1**
(43) Date of publication of application: **09.02.2011**
(21) Application number: 09178845.5
(22) Date of filing: 11.12.2009
(51) Int. Cl.: C07C 231/24, C07C 237/46, C07B 63/00, B01D 61/14

(54) **Recovering unreacted intermediate 5-acetamido-N,N'-bis(2,3-dihydroxypropyl)-2,4,6-triiodoisophthalamide from desalinated and desolventized dimerisation reaction mixture by ultrafiltration**

(30) Priority: 21.07.2009 US 227102 P
(71) Applicant: GE Healthcare AS, 0401 Oslo (NO)
(72) Inventor: Homestad, Ole, Magne, 4521 Spangereid (NO); Ingvoldstad, Odd, Einar, 4521 Spangereid (NO)
(74) Representative: Wulff, Marianne Weiby

(57) **Abstract**

This invention relates to a method of recovering intermediate 5-acetamido-N,N'-bis(2,3-dihydroxypropyl)-2,4,6-triiodoisophthalamide ("Compound A") from the desalinated and desolventized dimerisation reaction mixture of Compound A to iodixanol. In particular, the present invention employs ultrafiltration to recover non-crystalline Compound A to reduce the overall cost of iodixanol manufacture, increase the yield of iodixanol, and facilitate the subsequent purification procedures to meet the regulatory purity requirement of iodixanol, a non ionic X-ray contrasting agent.

## Description

### TECHNICAL FIELD

This invention relates generally to industrial preparation of iodixanol (1,3-bis(acetamido)-N,N'-bis[3,5-bis(2,3-dihydroxypropylaminocarbonyl)-2,4,6-triiodophenyl]-2-hydroxypropane), a non-ionic X-ray contrasting agent. It further relates to a method of recovering intermediate 5-acetamido-N,N'-bis(2,3-dihydroxypropyl)-2,4,6-triiodoisophthalamide ("Compound A") from a desalinated and desolventized dimerisation reaction mixture. In particular, the present invention employs ultrafiltration prior to the crystallisation of iodixanol to recover non-crystalline Compound A where the permeate contains less than about 8 % of Compound A by weight relative to iodixanol.

### BACKGROUND OF THE INVENTION

Iodixanol is the non-proprietary name of the chemical drug substance, 1,3-bis(acetamido)-N,N'-bis[3,5-bis(2,3-dihydroxypropylaminocarbonyl)-2,4 ,6-triiodophenyl]-2-hydroxypropane). Marketed under the trade name Visipaque®, iodixanol is one of the most used agents in diagnostic X-ray procedures. It is produced in large quantities by GE Healthcare in Lindesnes, Norway. The manufacture of iodixanol requires the production of the chemical drug substance (referred to as primary production) followed by formulation into the drug product (referred to as secondary production). The primary production of iodixanol involves a multistep chemical synthesis and a thorough purification process. It is important for the primary production to be efficient and economical and to provide a drug substance fulfilling the regulatory specifications, such as those mandated by US Pharmacopeia. In addition, the cost and efficiency of the secondary production depend on the synthesis and purification processes in the primary production. Thus, optimization is desired in each step of the primary production of iodixanol.

The industrial synthesis of iodixanol involves dimerisation of intermediate 5-acetamido-N,N'-bis(2,3-dihydroxypropyl)-2,4,6-triiodoisophthalamide ("Compound A") as the final synthetic step as shown in Scheme 1 below.

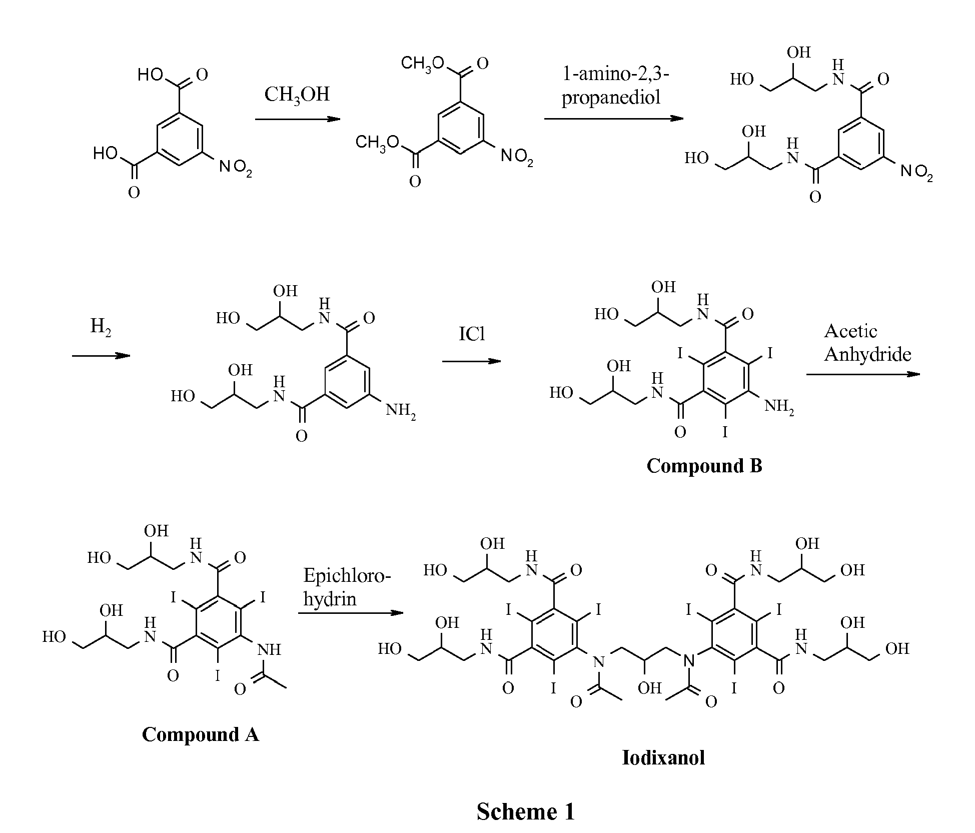

The dimerisation of Compound A to iodixanol leads to a conversion of about 55-60 % of the starting material. Most of the unreacted Compound A is subsequently removed and recovered from the reaction solution by addition of hydrochloric acid, which allows for precipitation of neutral Compound A from the reaction solution. *See* U.S. Patent No. 6,974,882. Despite the initial removal and recovery of Compound A, a considerable amount of Compound A, about 14-18 % relative to iodixanol, remains in the reaction solution. There exists a need for a cost-effective industrial process for the recovery of this additional Compound A.

### SUMMARY OF THE INVENTION

The present invention provides a process for recovering a key intermediate in the synthesis of iodixanol. It further relates to recycling residual non-crystalline Compound A before the initiation of the crystallisation process for iodixanol. Specifically, the instant invention is directed to the sequential steps of (1) reducing the salt and solvent content in a dimerisation reaction mixture containing Compound A and iodixanol to precipitate Compound A in a non-crystalline form; (2) passing the mixture of step (1) through an ultrafiltration membrane; (3) recovering non-crystalline Compound A in the retentate of step (2) for reuse in a subsequent dimerisation reaction to prepare iodixanol; and (4) crystallising iodixanol from the permeate of step (2) wherein the weight content of Compound A in the permeate is less than about 8% relative to that of iodixanol. A suitable ultrafiltration system may include tubular, spiral or hollow fibre based systems.

The present procedure represents an optimal industrial process. In particular, the wastage of Compound A is minimized without adding excessive time or incurring substantial cost. In addition, the iodixanol solution before its crystallisation contains sufficiently less Compound A to enable the final iodixanol product to meet the regulatory requirement.

### DETAILED DESCRIPTION OF THE INVENTION

Following the acid quenching of the dimerisation reaction from Compound A to iodixanol and the initial recovery of unreacted Compound A by precipitation, about 14-18 % of Compound A (relative to iodixanol by weight) is still dissolved in solution. The dissolved Compound A, along with the iodixanol product in solution, could be fed to the next process step without any discrete recovery step for Compound A. This procedure however presents several problems.

First, the unrecovered Compound A represents a loss of valuable intermediate in the primary production of iodixanol. This waste of Compound A is significant because it is the last intermediate in the iodixanol manufacture and because it contains iodine, the most expensive reagent in the chemical synthesis. Any loss of Compound A increases the overall cost of the primary production of iodixanol.

Second, Compound A can be reused in a subsequent dimerisation reaction to prepare iodixanol. In other words, the additional Compound A in solution with iodixanol can be converted to iodixanol in a new dimerisation reaction. The unrecovered Compound A thus lowers the effective production yield of iodixanol.

Finally, we have found that a high content of Compound A in the feed to subsequent crystallisation steps of iodixanol makes it difficult to obtain the required purity of the final iodixanol product.

To address these issues, an effective and efficient method has been found to recover the additional soluble Compound A. Specifically, the reduction of salt content of the dimerisation reaction mixture causes the solubility of Compound A to decrease, which leads to the precipitation of Compound A. In addition, the amount of solvent is reduced along with the salt content, which again contributes to the precipitation of Compound A due to its poor solubility in water.

We have found that the precipitated Compound A during the salt and solvent reduction process is largely non-crystalline. However, conventional filtration techniques, such as pressure or vacuum filtration, are not suitable for an industrial scale recovery of Compound A due to a variety of factors, such as the added cost and time, the compatibility with the existing iodixanol primary production operation, and the loss of iodixanol.

On the other hand, the additional Compound A precipitated during desalination and desolventization can be efficiently removed by ultrafiltration with minimal addition of time and cost. Further, the ultrafiltration cake can be combined with the precipitated Compound A from the previous hydrochloric acid precipitation step. Pooling Compound A from two separate recovery steps gives a net yield increase in the process of primary production of iodixanol and enhances the economy of production considerably.

Another improvement of the instant process is that the content of Compound A in the process solution subjected to iodixanol crystallisation is reduced to a level that the residual Compound A in the ultrafiltration permeate does not interfere with the subsequent crystallisation of iodixanol. In certain embodiments, the level of compound A in the crystallisation feed is between about 4 and about 8 w/w % relative to iodixanol. It has been found that this relative small amount of Compound A left in the permeate solution containing iodixanol after ultrafiltration can be removed in the crystallisation process for iodixanol without the need for expensive and time consuming reprocessing steps.

Yet another improvement of the instant process is that the loss of the main product iodixanol is kept at a minimal during ultrafiltration. The solubility of iodixanol in water has been found to be high enough such that it does not precipitate during the instant process of recovering additional Compound A.

The invention is illustrated further by the following examples that are not to be construed as limiting the invention in scope to the specific procedures described in them.

### EXAMPLES

### EXAMPLE 1

A reaction mixture containing about 340 kg iodixanol and substantial amounts of Compound A (about 14-18 w/w % relative to iodixanol) and iohexol (6-8 w/w % relative to iodixanol) is subjected to nanofiltration. Water is added continuously to facilitate diafiltration followed by volume reduction. A final salt concentration of about 0.60 w/w % relative to iodixanol (2.0 kg NaCl in 340 kg iodixanol) is obtained. At this stage, the reaction medium is aqueous with the pH between about 4 and 6. Compound A is precipitated on the retentate side of the nanofiltration membrane due to reduced salt and organic solvent content. The organic solvent is 2-methoxyethanol.

The precipitated Compound A is removed from the process solution by ultrafiltration using a Pallsep™ PS400 vibrating membrane system at ambient temperature with the pH between about 5 and 7.5. At the end of the ultrafiltration step water is added continuously to facilitate diafiltration in order to flush out any remaining iodixanol on the retentate side. The diafiltration step is terminated when almost pure water flows through the ultrafilter, detected by a density of the permeate of less than 1.005 kg/L. The last fraction of permeate is led to a different stream than the main process solution for later re-use in an earlier step to avoid dilution of the product mixture before crystallisation. The Compound A content in the main filtrate is about 4 to about 7 % (w/w) relative to iodixanol content. The filtrate is subjected to crystallisation and subsequent purification steps to obtain the necessary purity.

### EXAMPLE 2

A reaction mixture containing about 340 kg iodixanol and substantial amounts of Compound A (about 14-18 w/w % relative to iodixanol) and iohexol (6-8 w/w % relative to iodixanol) is subjected to nanofiltration. Water is added continuously to facilitate diafiltration followed by volume reduction. A final salt concentration of about 0.60 w/w % relative to iodixanol (2.0 kg NaCl in 340 kg iodixanol) is obtained. At this stage, the reaction medium is aqueous with the pH between about 4 and 6. Compound A is precipitated on the retentate side of the nanofiltration membrane due to reduced salt and organic solvent content. The organic solvent is methanol.

The precipitated Compound A is removed from the process solution by ultrafiltration using a Pallsep™ PS400 vibrating membrane system at ambient temperature with the pH between about 5 and 7.5. At the end of the ultrafiltration step water is added continuously to facilitate diafiltration in order to flush out any remaining iodixanol on the retentate side. The diafiltration step is terminated when almost pure water flows through the ultrafilter, detected by a density of the permeate of less than 1.005 kg/L. The last fraction of permeate is led to a different stream than the main process solution for later re-use in an earlier step to avoid dilution of the product mixture before crystallisation. The Compound A content in the main filtrate is about 4 to about 7 % (w/w) relative to iodixanol content. The filtrate is subjected to crystallisation and subsequent purification steps to obtain the necessary purity.

All patents, journal articles, publications and other documents discussed and/or cited above are hereby incorporated by reference.

## Claims

1. A process for recovering 5-acetamido-N,N'-bis(2,3-dihydroxypropyl)-2,4,6-triiodoisophthalamide ("Compound A") after desalination and desolventization of a dimerisation reaction mixture of Compound A to iodixanol comprising the sequential steps of:
(1) reducing salt and solvent content in the dimerisation reaction mixture containing Compound A and iodixanol to precipitate Compound A in a non-crystalline form;
(2) passing the mixture of step (1) through an ultrafiltration membrane;
(3) recovering non-crystalline Compound A in the retentate of step (2) for reuse in a subsequent dimerisation reaction to prepare iodixanol; and
(4) crystallising iodixanol from the permeate of step (2) wherein the weight content of Compound A in the permeate is less than about 8 % relative to that of iodixanol.
